# EUROPEAN PATENT APPLICATION

(11) **EP 4 063 488 A1**
(43) Date of publication of application: **28.09.2022**
(21) Application number: 20890370.8
(22) Date of filing: 20.11.2020
(51) Int. Cl.: C12N 5/0775, A61K 35/76, A61K 35/761

(54) **MESENCHYMAL STEM CELLS FOR USE AS VEHICLES FOR THERAPEUTIC AGENTS**

(30) Priority: 21.11.2019 ES 201931029
(71) Applicant: Fundación Para La Investigación Biomédica Del Hospital Infantil Universitario Niño Jesús, 28009 Madrid (ES); Fundación Oncohematología Infantil, 28015 Madrid (ES)
(72) Inventor: RAMÍREZ ORELLANA, Manuel, 28009 Madrid (ES); FRANCO LUZÓN, Lidia, 28015 Madrid (ES); GONZÁLEZ MURILLO, África, 28009 Madrid (ES); MELEN FRAIJLICH, Gustavo Javier, 28009 Madrid (ES)
(74) Representative: Pearl Cohen Zedek Latzer Baratz UK LLP
(86) International application number: PCT/ES2020/070726
(87) International publication number: WO 2021/099671

(57) **Abstract**

Mesenchymal stem cells for use as vehicles for therapeutic agents. The present invention is related to a method for selecting or obtaining mesenchymal stem cells (MSCs) that can be used as transporters or vehicles for therapeutic agents, especially in cancer treatment.

## Description

### FIELD OF THE INVENTION

The present invention may be included in the field of advanced therapies, that is, cell therapy, gene therapy or virotherapy. In particular, the present invention is related to a method for selecting or obtaining mesenchymal stem cells (MSCs) that can be used as transporters or vehicles for therapeutic agents, especially in cancer treatment.

### PRIOR ART

Virotherapy, gene therapy or cell therapy are often applied in oncology with the object of using MSCs as transporters or vehicles for therapeutic agents which act selectively on the tumor. In fact, various strategies exist directed at modifying or adapting said MSCs with the object of boosting their therapeutic capacity in the treatment of cancer. For example, MSCs may be infected with a conditionally replicating oncolytic virus which applies its antitumor activity selectively on the tumor. Alternatively, the MSCs may have genetic modifications with the object of producing therapeutic proteins for the treatment of cancer. However, said MSCs may incorporate active ingredients by means of nanoparticles or may even exhibit surface changes to improve their therapeutic efficacy.

One of the most innovative strategies in this field is the use of oncolytic viruses that act as therapeutic agents to eliminate or reverse the development of a cancerous tumor in the organism. The use of these viruses for therapeutic purposes is known as virotherapy. Virotherapy uses viruses specifically designed to selectively eliminate tumor cells. Unlike conventional drugs, these viruses are multiplied selectively in the tumor, and therefore efficacy can be very high from a very low initial dose.

In recent years, various therapeutic projects and strategies have been developed based on virotherapy. Outstanding within the field of virotherapy for the treatment of tumors, particularly metastatic and refractory pediatric tumors, is the product known as Celyvir. Celyvir is made up of MSCs from the patient, which are transfected with a conditionally replicating oncolytic adenovirus. The MSCs are capable of detecting and moving to the tumors, depositing in the interior thereof the oncolytic virus responsible for destroying said tumor cells there. Moreover, the viral infection also activates the immune system which, in some patients, triggers a response from their own immune system to the tumor, destroying said tumor.

In particular, Celyvior comprises autologous MSCs transfected with the ICOVIR-5 virus. ICOVIR-5 (HAd5-DM-E2F-K-Δ24-RGD) is an oncolytic adenovirus derived from HAd5 and designed for the systemic treatment of disseminated tumors. The ICOVIR-5 genome contains various modifications giving it the ability to replicate selectively in tumor cells in which the retinoblastoma/E2F pathway is deregulated. The different genetic modifications contained in ICOVIR-5 are:
i) Substitution of part of the endogenous E1A promoter by the E2F-1 promoter to control the expression of the E1A viral protein (GenBank S74230). The E2F1 promoter is a non-coding sequence of human origin. It is obtained by PCR from DNA obtained from normal human peripheral blood mononuclear cells using oligonucleotides which amplified the region between positions - 218 and +51 of the E2F-1 RGD gene sequence.
ii) Insertion of an isolate sequence (DM) preceding the E2F-1 promoter. The DM-1 isolate comes from the human DM1 locus. It is obtained by PCR from DNA obtained from normal human peripheral blood mononuclear cells, using oligonucleotides which amplified the region between positions 13006 and 13474 of the sequence of the DM-1 genetic locus (GenBank access number L08835). It isolates the E2f1 promoter in the viral genome.
iii) Insertion of the Kozak sequence (CCACC) in the initiation E1A codon (K). This sequence facilitates recognition of the mRNA translation machinery.
iv) The presence of a deletion of 8 amino acids in the binding site of E1A with pRb (Δ24 deletion).
v) Insertion of the tripeptide Arg-Gly-Asp (RGD) in the HI loop in the viral fiber. Inserting the DGD peptide in the HI loop increases the replicating power of the virus.

Celyvir has shown very positive results, even leading to a cure in cases of infantile neuroblastomas. However, the reason why some patients respond positively to the treatment and other patients do not respond is still unknown. Studies exist that indicate that the activity of the oncolytic virus may be inhibited by the patient's own immune system thus inhibiting its therapeutic effect.

It would therefore be very positive to be able to develop a method aimed at selecting, obtaining or producing MSCs that are capable of effectively delivering therapeutic agents in order to apply their anticancer action selectively, without being affected or inhibited by the patient's own immune system.

The present invention is aimed at overcoming the above-mentioned technical problem as it relates specifically to a method for selecting, obtaining or producing MSCs that can be used as transporters or vehicles for therapeutic agents, especially in the treatment of cancer.

### DESCRIPTION OF THE INVENTION

### Brief description of the invention

After spending years studying the outcomes of virotherapy treatment in children with solid tumors, the researchers of the present invention reached the conclusion that one of the key aspects to ensure that virotherapy used for the treatment of cancer is really effective is the existence of populations of MSCs that are particularly capable of hiding and protecting the oncolytic adenovirus from recognition and elimination by the patient's own immune system, thus making it easier for the oncolytic virus to reach the metastases and enhancing its antitumor capacity.

Thus, starting from said hypothesis, the conclusion was reached in the present invention that an optimal molecular profile exists in some MSCs that makes them particularly capable of hiding and protecting the oncolytic adenovirus from immune recognition and elimination, making it easier for said oncolytic virus to reach the metastases and increasing its antitumor capacity.

As there are patients with positive clinical responses to Celyvir and others who do not show a response to said treatment, comparing the molecular profiles of the MSCs of these two groups of patients is used to identify the molecules that are responsible for hiding and protecting the oncolytic virus, leading to a more effective therapeutic response.

Thus, as shown in the present invention, patients who responded to the treatment had been treated with MSCs of which the mitochondrial antiviral-signaling protein (MAVS) gene [Gene ID: 57506] was inhibited, and showed a significantly lower gene expression than non-responder patients.

The first aspect of the present invention therefore relates to a substantially pure population of MSCs, wherein the MSCs constitute at least 80% of the total cell population, characterized in that expression of the MAVS gene (or other genes regulated by said gene) is inhibited, or in that the MAVS protein is not expressed. Among the genes of which the expression is regulated by MAVS are, for example, IL6 and CCL2.

In a preferred aspect, the MSCs are derived from bone marrow, the placenta, umbilical cord, amniotic membrane, menstrual blood, peripheral blood, salivary gland, skin and prepuce, synovial fluid, amniotic fluid, endometrium, adipose tissue, umbilical cord blood and/or dental tissue.

In a preferred aspect, the MSCs are autologous or allogeneic.

The second aspect of the invention relates to said mesenchymal stem cells characterized in that expression of the MAVS gene (or other genes regulated by said gene) is inhibited, or in that the MAVS protein is not expressed, for use as transporters or vehicles for therapeutic agents.

The third aspect of the invention relates to a composition that comprises: i) at least one cell characterized in that expression of the MAVS gene (or other genes regulated by said gene) is inhibited, or in that the MAVS protein is not expressed, and ii) at least one antigenic substance.

The fourth aspect of the invention relates to a pharmaceutical composition that comprises: i) at least one cell characterized in that expression of the MAVS gene (or other genes regulated by said gene) is inhibited, or the MAVS protein is not expressed, ii) at least one antigenic substance and, optionally, pharmaceutically acceptable vehicles, adjuvants and/or excipients.

In a preferred aspect, the antigenic substance is an intracellular parasite.

In a preferred aspect, the intracellular parasite is an oncolytic virus.

In a preferred aspect, the intracellular parasite is an oncolytic adenovirus.

In a preferred aspect, the intracellular parasite is ICOVIR-5.

In a preferred aspect, said pharmaceutical composition is used as a medicine, preferably in the treatment of solid tumors, particularly brain cancer, lung cancer, renal cancer, ovarian cancer and/or liver cancer, bone cancer, neuroblastoma, rhabdomyosarcoma, Ewing sarcoma.

The fifth aspect of the invention relates to an *in vitro* method of producing (manufacturing) a substantially pure population of modified mesenchymal stem cells which comprises:
a. Obtaining mesenchymal stem cells from an isolated sample;
b. Culturing the mesenchymal stem cells;
c. Inhibiting expression of the MAVS gene in the cultured cells.

In a preferred aspect, the method also comprises infecting the cells with an intracellular parasite, preferably an oncolytic virus, particularly ICOVIR-5.

The sixth aspect of the present invention relates to a kit that comprises: a) at least one mesenchymal stem cell; and b) a system capable of inhibiting expression of the MAVS gene (or other genes regulated by said gene).

In a preferred aspect of the invention, said substantially pure population of MSCs is selected from the MSCs present in a biological sample obtained from the patient, selecting the MSCs that express low levels of the MAVS gene. Alternatively, in a preferred aspect of the invention, the MSCs are genetically modified to obtain MSCs directly that do not express MAVS protein, without having to make a selection of MSCs from a biological sample. The MSCs may be genetically modified using any type of genetic engineering that serves to block, inhibit or reduce the expression of the MAVS gene/protein. Examples of said techniques may include RNA interference, reverse oligonucleotide, or CRISPR technology, etc.

In a preferred aspect of the invention, the substantially pure population of MSCs is characterized in that said cells are infected with a conditionally replicating oncolytic virus. In a particular embodiment, the conditionally replicating oncolytic virus is an adenovirus which comprises a human DNA sequence derived from the myotonic dystrophy locus isolating a promoter that confers selective expression on an adenoviral gene. In a particular embodiment, the conditionally replicating oncolytic virus is an adenovirus that comprises the E2F1 human gene promoter that confers tumor selectivity. In a particular embodiment, the conditionally replicating oncolytic virus is an adenovirus that comprises a Kozak sequence that optimizes the protein translation of an adenoviral gene regulated by a promoter that confers tumor selectivity. In a particular embodiment, the conditionally replicating oncolytic virus is an adenovirus that comprises mutations in one or more genes of the E1a, E1b and E4 group to achieve selective replication in tumors. In a particular embodiment, the conditionally replicating oncolytic virus is an adenovirus that comprises the RGD tripeptide in the HI loop in the viral fiber.

In a preferred aspect of the invention, the substantially pure population of MSCs is characterized in that said cells have genetic modifications with the object of producing therapeutic proteins. In a particular embodiment, the substantially pure population of MSCs is characterized in that the genetic modifications of the cells takes place by transduction with viral vectors selected from the group that consists in retroviruses, lentiviruses, baculoviruses or adeno-associated viruses. In another particular embodiment, the substantially pure population of MSCs is characterized in that genetic modifications of the cells are achieved by non-viral transfection using physical methods selected from the group that consists in electroporation, nucleofection and sonoporation or chemical methods selected from the group that consists in lipidemic agents, polymeric carriers, dendrimers and inorganic nanoparticles.

In a preferred aspect of the invention, the substantially pure population of MSCs incorporates therapeutic agents. In a preferred embodiment, the substantially pure population of MSCs is characterized in that it incorporates non-peptide therapeutic agents and nucleic acids. In another preferred embodiment, the substantially pure population of MSCs is characterized in that the non-peptide therapeutic agents and nucleic acids are incorporated in the cells by means of polymeric or liposome nanoparticles by cell internalization or binding to the cell surface.

In a preferred aspect of the invention, the substantially pure population of MSCs shows modifications to their surface to improve their therapeutic efficacy. In a particular embodiment, the substantially pure population of MSCs is characterized in that the modification of the surface is an enzymatic modification which comprises the cell functionalization with adhesive ligands. In another particular embodiment, the substantially pure population of MSCs is characterized in that surface modification is a chemical modification which comprises the covalent conjugation of the cells with ligands, peptides or polymers. In another particular embodiment, the substantially pure population of MSCs is characterized in that surface modification is a non-covalent modification wherein the cells are conjugated with antibodies or peptides.

To clarify the present invention, the following definitions are included:
- Throughout the present invention, the term "mesenchymal stem cells (MSCs)" refers to a multipotent stromal cell originating from the mesodermal germinal layer, which can be differentiated into different cell types, including osteocytes (bone cells), chondrocytes (cartilage cells) and adipocytes (fat cells). The markers expressed by the MSCs include CD105 (SH2), CD73 (SH3/4), CD44, CD90 (Thy-1), CD71 and Stro-1 as well as the CD106, CD166 and CD29 adhesion molecules. Negative markers for MSC (not expressed) include, among others, the CD45, CD34, CD14 hematopoietic markers and the CD80, CD86 and CD40 co-stimulatory molecules, as well as the CD32 adhesion molecule. The MSCs may be obtained, without limitation, from bone marrow, adipose tissue (such as subcutaneous adipose tissue), liver, spleen, testicles, menstrual blood, amniotic fluid, pancreas, periosteum, synovial membrane, skeletal muscle, dermis, pericytes, trabecular bone, human umbilical cord, lung, dental pulp and peripheral blood. The MSCs according to the invention may be obtained from any of the preceding tissues, such as bone marrow, subcutaneous adipose tissue or umbilical cord. The MSCs may be isolated from bone marrow by methods known to persons skilled in the art. Said methods generally consist in isolating mononuclear cells by density gradient centrifugation (Ficoll, Percoll) of bone marrow aspirates, and then seeding the isolated cells on tissue culture plates in a medium containing fetal bovine serum. These methods are based on the ability of the MSCs to adhere to plastic, so that while the non-adherent cells are eliminated from the culture, the adherent MSCs can be grown on the culture plates. The MSCs can also be isolated from subcutaneous adipose tissue following a similar method known to persons skilled in the art. A method for isolating bone marrow or subcutaneous adipose tissue MSCs has been described previously (De la Fuente et al., Exp. Cell Res. 2004, Vol. 297:313:328).
- Throughout the present invention, the term "substantially pure population" refers to a cell population in which MSCs constitute at least 80% of the total cells in the population, preferably at least 85, 90, 95, 96, 97, 98 or 99% of the total cells in the population. The composition according to the invention may therefore comprise a cell population in which at least approximately 25%, at least approximately 30%, at least approximately 35%, at least approximately 40%, at least approximately 45%, at least approximately 50%, at least approximately 55%, at least approximately 60%, at least approximately 65%, at least approximately 70%, at least approximately 75%, at least approximately 80%, at least approximately 85%, at least approximately 90%, at least approximately 95%, at least approximately 96%, at least approximately 97%, at least approximately 98%, or at least approximately 99% of the cells are MSCs. In other words, in some embodiments at least approximately 25%, at least approximately 30%, at least approximately 35%, at least approximately 40%, at least approximately 45%, at least approximately 50%, at least approximately 55%, at least approximately 60%, at least approximately 65%, at least approximately 70%, at least approximately 75%, at least approximately 80%, at least approximately 85%, at least approximately 90%, at least approximately 95%, at least approximately 96%, at least approximately 97%, at least approximately 98%, or at least approximately 99% of the cells in the composition are MSCs. The composition according to the invention may comprise at least approximately 25%, at least approximately 30%, at least approximately 35%, at least approximately 40%, at least approximately 45%, at least approximately 50%, at least approximately 55%, at least approximately 60%, at least approximately 65%, at least approximately 70%, at least approximately 75%, at least approximately 80%, at least approximately 85%, at least approximately 90%, at least approximately 95%, at least approximately 96%, at least approximately 97%, at least approximately 98%, or at least approximately 99% MSCs, calculated by number, by weight or by volume of the composition.
- The term "pharmaceutical composition" refers to a composition intended for use in therapy. The compositions according to the invention are pharmaceutical compositions intended for use in regenerative medicine or cell therapy for the treatment of cancer. The compositions according to the invention may include, as well as the populations described in the present invention, non-cellular components. Examples of such non-cellular components include, but are not limited to, cell culture mediums, which may comprise one or more proteins, amino acids, nucleic acids, nucleotides, coenzymes, antioxidants and metals.
- The expression "pharmaceutically acceptable" is used in the present document to refer to those compounds, materials, compositions and/or dosage forms that, within the limits of good medical judgement, are suitable for use in contact with human and animal tissues without excessive toxicity, irritation, allergic reaction or any other problem or complication, proportional to a reasonable risk/benefit relationship.
- As used used in this document, the expression "pharmaceutically acceptable vehicle" means a pharmaceutically acceptable material, composition or vehicle, such as a filler material, diluent, excipient, or liquid or solid solvent involved in transporting or transporting the object compound from one organ or part of the body to another organ or part of the body. A vehicle must be "acceptable" in the sense of being compatible with other components of the formulation and not causing harm to the patient.
- The term "which comprises" means it includes, but is not limited to, what follows the words "which comprises." Thus, the use of the term "which comprises" indicates that the elements listed are mandatory or mandatory, but that other elements are optional and may or may not be present.
- "Which consists in" is understood to mean it includes, and is limited to, what follows the phrase "which consists in." Thus, the phrase "which consists in" indicates that the elements listed are mandatory, and that other elements may not be present.
- "Therapeutic agent" is understood in the present invention to mean any chemical or biological molecule capable of having a therapeutic effect in the treatment of cancer, for example, oncolytic viruses, active protein or non-protein ingredients, etc.

### Brief description of the figures

**Fig. 1****.** Diagram illustrating the process of obtaining the MSCs according to the invention, characterized in that expression of the MAVS gene (or other genes regulated by said gene) is inhibited, or in that the MAVS protein is not expressed.
**Fig. 2****.** Relative expression of the differentially expressed genes by ANN analysis for the immune response to the virus process (MAVS, p ≤ 0.001; NDRG1, p ≤ 0.0116).
**Fig. 3****.** Quantification of the proteins differentially secreted in the supernatants of the Celyvir cultures. Of the five proteins analyzed, only IL-6 and MCP-1 were detected with the multiplex assays used (p ≤ 0.05).
**Fig. 4****.** Graphic illustration of the proposed model.

### Detailed description of the invention

### Example 1. Materials and methods

### Example 1.1. Design of the study

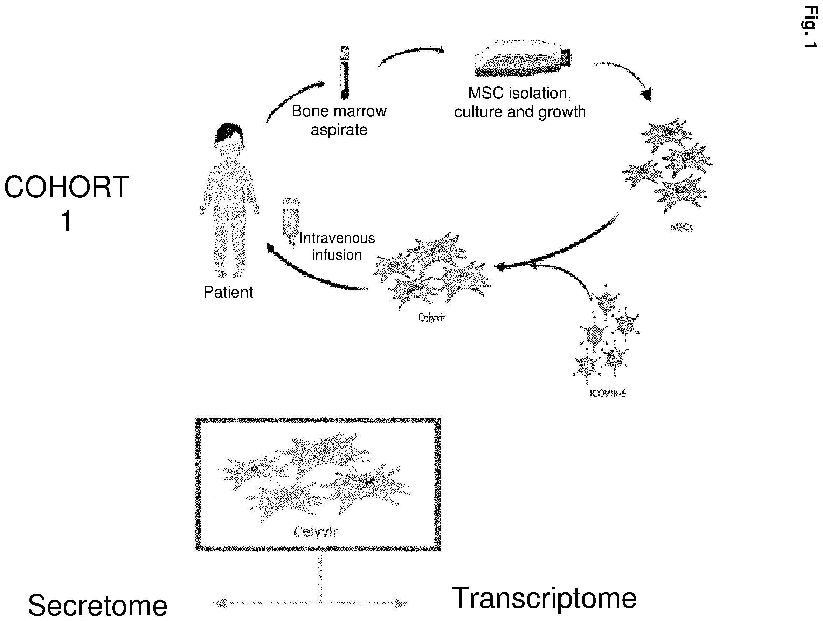

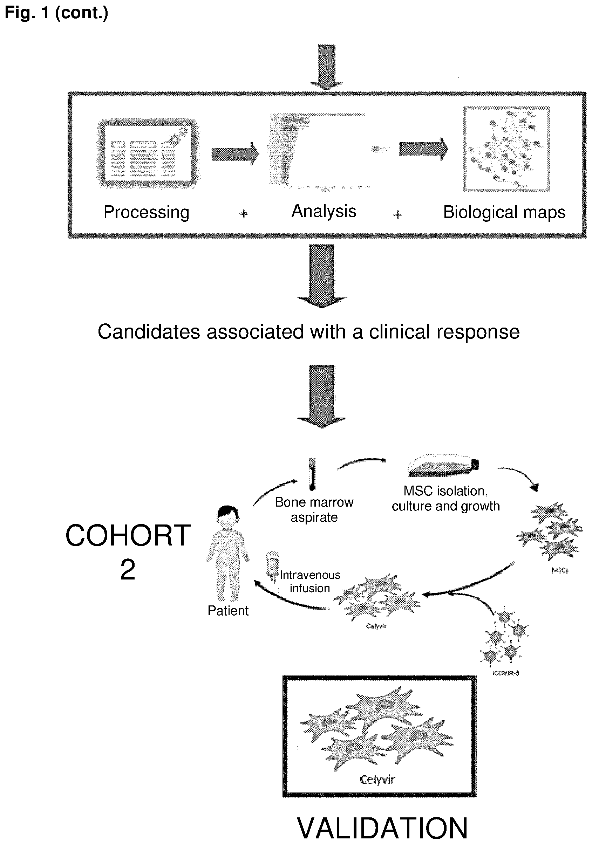

Celyvir is an advanced therapy medicinal product (ATMP) produced with autologous mesenchymal cells and the oncolytic adenovirus ICOVIR-5. Variability is associated with the individual and intrinsic characteristics of the cells of the ATMP which could have an impact on the quality of the medicine, which in turn may affect the clinical response. These biological characteristics in the different Celyvir products administered to the first cohort of patients were studied exhaustively, as shown in **Fig. 1****.** Using an approximate analysis of biological systems, biological programs were identified that functioned differently between patients who responded to the treatment and those who did not. Moreover, molecules responsible for said differences were identified. These candidates were validated in a second cohort of patients treated with Celyvir.

### Example 1.2. Obtaining mesenchymal cells from patients

The MSCs were obtained by aspirating bone marrow from the iliac crest of the patients. Mononucleated cells were obtained by Ficoll gradient centrifugation (Ficoll-Paque^{™} PLUS, GE Healthcare) at 1500 rpm for 20 minutes at 20°C. The layer of mononuclear cells obtained after centrifugation were isolated from the rest of the components of the gradient using a Pasteur pipette. The cells were transferred to a clean Falcon tube and washed with phosphate buffered saline (PBS), Gibco. After centrifuging for one last time at 1500 rpm for 5 minutes, the mononucleated cells were resuspended in culture medium.

### Example 1.3. Culture of the mesenchymal cells from patients

The initial mononucleated fraction was cultured at a density of 500,000 cells/cm2 of surface with Dulbecco's Modified Eagle Medium (DMEM 1g/L glucose, Gibco, Carlsbad, CA), supplemented with 10% fetal bovine serum (FBS, HyClone, Logan, UT) which had previously been inactivated in a wet bath for 20 minutes at 56°C, and 1% penicillin-streptomycin (P/S, Gibco). The culture medium was replaced 48 hours later, which was sufficient time for the MSCs to adhere to the plastic while the rest of the mononucleated cells remained in suspension in the culture medium. After this first change, the medium was replaced every 3-5 days.

The MSCs were kept in an atmosphere at 37°C and 5% CO₂ until reaching an adequate confluency level (approximately 80%). At the moment of trypsinization, the cells were washed once with PBS and then trypsin was added thereto (TrypLE Express, Life Technologies, Carlsbad, CA). The cells that had detached from the plastic surface were collected using fresh culture medium and a pipette, and centrifuged at 1500 rpm for 5 minutes to remove the trypsin. The MSC pellet obtained was resuspended in fresh culture medium, and the cells were re-seeded at 3000-5000 cells/cm2.

In the third or fourth cell pass, the MSCs were characterized by flow cytometry.

### Example 1.4. Icovir-5 oncolytic adenovirus

The characteristics of ICOVIR-5 have been described by its inventors (Alonso MM, Cascallo M, Gomez-Manzano C, Jiang H, Bekele BN, Perez-Gimenez A et al. ICOVIR-5 shows E2F1 addiction and potent antiglioma effect in vivo. Cancer Res 2007; 67: 8255-8263). For this study, it was produced in the Vector Production Unit of the University of Barcelona.

### Example 1.5. Celyvir preparation

The preparation of the medicine for human use met good manufacturing practice (GMP) standards and the administration thereof to patients has already been described by the present team (Melen GJ, Franco-Luzón L, Ruano D, Gonzalez-Murillo Á, Alfranca A, Casco F et al. Influence of carrier cells on the clinical outcome of children with neuroblastoma treated with high dose of oncolytic adenovirus delivered in mesenchymal stem cells. Cancer Lett 2016; 371: 161-170).

### Example 1.6. Celyvir transcriptome study (RNA sequencing)

For the transcriptome study, the cell fraction of the Celyvir cultures was used from which the supernatants were also removed. The RNA was extracted using the Absolutely RNA Microprep Kit (Agilent, Santa Clara, CA) and each sample was treated with DNasa to avoid DNA contamination. 3 µg of RNA from each sample were sent to the Massive Sequencing Unit at the Madrid Science Park where massive sequencing was carried out. The raw data obtained were used for the subsequent statistical analysis.

### Example 1.7. Validation of the results obtained in the transcriptome analyses

### Validation of the transcriptome results using qPCR

To validate the candidate genes identified by the bioinformatics analyses, a quantitative PCR was carried out using TaqMan technology (Applied Biosystems, Foster City, CA) on Celyvir samples from a cohort of patients that was independent of the initial cohort used during the identification process (responders, n=5; non-responders, n=10).

**Table 1** shows the Applied Biosystems assays used.

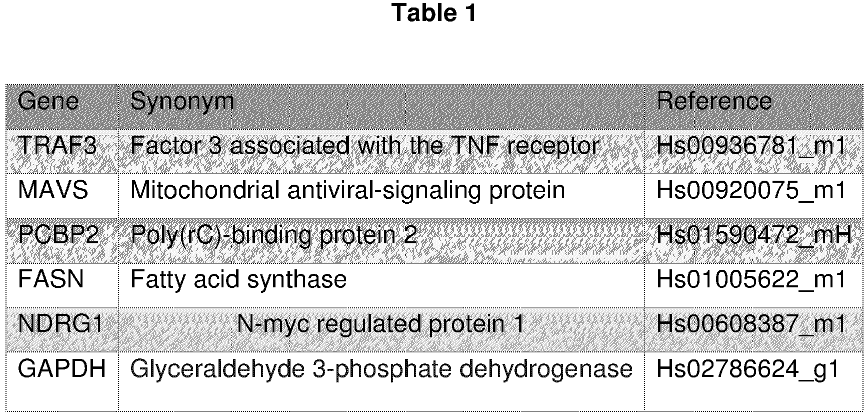

The qPCR reaction was carried out using TaqMan Gene Expression Master Mix (Applied Biosystems, Foster City, CA) and the proportion of the elements for a PCR reaction is that shown in **Table 2** for a final volume of 20 µL:

**Table 2**

| | |
|---|---|
| *TaqMan Gene Expression Master Mix (2X)* | 10 µL |
| *TaqMan Gene Expression Assay (20X)* | 1 µL |
| *RNAsa-free water* | 4 µL |
| *cDNA* | 5 µL |
| ***FINAL REACTION VOLUME*** | **20 µL** |

### Quantification of secretome candidates bv Luminex

To functionally validate the results of the genes determined in the previous paragraph, the levels of some proteins in the Celyvir supernatants from a cohort of patients independent of the initial cohort used during the identification process (responders, n=5; non-responders, n=10) were quantified using Luminex xMAP technology. To do this, the following Luminex xMAP assays were used, in accordance with the instructions of the manufacturer:
- MILLIPLEX MAP Human Cytokine/Chemokine Magnetic Bead Panel (HCYTOMAG-60K, Merck, EMD Millipore Corporation, Billerica, MA) for measuring interleukin 4 (IL-4), interleukin 6 (IL-6), interleukin 10 (IL-10) and MCP-1 (CCL2).
- MILLIPLEX MAP Human Soluble Cytokine Receptor Panel (HSCRMAG-32K, Merck, EMD Millipore Corporation, Billerica, MA) for measuring the interleukin 6 receptor 6 (IL-6R).

The plates were analyzed using MAGPIX^{®} equipment (EMD Millipore Corporation, Billerica, MA) and the results were interpreted using MAGPIX xPONENT software (EMD Millipore Corporation, Billerica, MA).

### Example 2. Results

### Example 2.1. Transcriptome analysis

The data from the transcriptome were processed using conventional statistical analyses in order to identify genes with greater differential expression between the MSCs of responders and non-responders. In a first approximation, two groups of genes were considered: those that showed significant statistical enrichment with a level of error of 1% (p < 0.01) and those to which a less restrictive criterion (p < 0.05) had been applied in order to produce a more extensive list of potentially interesting results. Three statistical tests were carried out (ANOVA, Wilcoxon and T-student) to evaluate statistically significant differences in gene expression between responder and non-responder patients. The following positive results were obtained following the corrected multitest (FDR < 0.05): only 15 results, and only those derived from the ANOVA test. **Table 3** shows the candidate genes that in a first approximation showed significant differences between the responder and non-responder patient groups.

**Table 3**

| Uniprot ID | Gene name | Secretome | ANOVA FDR q-value | Log₂ FC | FC |
|---|---|---|---|---|---|
| P35573 | AGL | No | 0.01280 | 6.49492 | 90.19145 |
| P09238 | MMP10 | Yes | 0.01280 | 8.65044 | 401.83075 |
| Q12834 | CDC20 | No | 0.01280 | -8.88274 | 0.00212 |
| P11597 | CETP | No | 0.01280 | -9.02274 | 0.00192 |
| P19404 | NDUFV2 | No | 0.01280 | -10.89015 | 0.00053 |
| P16284 | PECAM1 | No | 0.01280 | -7.35809 | 0.00610 |
| A2AJT9 | CXorf23 | No | 0.01280 | -6.79375 | 0.00901 |
| P17021 | ZNF17 | No | 0.02209 | -9.33322 | 0.00155 |
| Q8IWZ8 | SUGP1 | No | 0.02236 | 8.37531 | 332.06250 |
| Q96Q89 | KIF20B | No | 0.02236 | 6.68242 | 102.70893 |
| Q8TE49 | OTUD7A | No | 0.02236 | -8.93858 | 0.00204 |
| Q92990 | GLMN | No | 0.02236 | -8.50728 | 0.00263 |
| Q9NZH4 | PTTG3P | No | 0.02236 | -10.71427 | 0.00060 |
| O60930 | RNASEH1 | No | 0.04146 | 8.51271 | 365.24350 |
| O75153 | CLUH | No | 0.04974 | -1.65012 | 0.31861 |

Being less statistically restrictive, a total of 113 genes showed a significant uncorrected p-value (p-value < 0.01) for at least one of the three tests carried out.

### Example 2.2. Gene set enrichment analysis (GSEA)

The above data were uploaded to the GSEA platform to identify the differential molecular pathways and processes between the MSCs of patients who responded to therapy using Celyvir and those who did not. This approximation provided an ordered list of genes where the more significant the difference, the higher the position in said list. In this case, the parameter used to classify said genes was the ratio between the signal emitted and the background signal for each sample. Those that had a background signal in all the samples were excluded from the GSEA. With these criteria, the expression levels of 13,715 genes in the signal-noise ratio parameter were transformed. A total of 5601 genes were left out of the analysis as no signal was detected in any of the samples.

81 significantly enriched pathways were found for the cohort of patients who responded to Celyvir, and 59 pathways for the non-responder patient cohort.

The most relevant processes and pathways provided by GSEA were:
*1. **Viral infection and metabolism of nucleic acids.*** The GSEA results indicated that the expression of the genes involved in these processes was increased in the cohort of responder patients compared with non-responders. Some processes relating to nucleic acids appeared as representative of the cohort of responder patients, including groups of genes relating to mechanisms for regulating deoxyribonucleic acid (DNA) transcription, DNA replication, DNA-ribonucleic acid (RNA) interaction, ribosomal RNA, RNA splicing and translation. In addition, groups of genes directly relating to viral biology (positive regulation of viral release by the host cell, viral life cycle) appear overexpressed in the samples of responder patients.
2. ***Processes relating to the immune response.*** These processes appeared increased in the MSCs of both cohorts of patients. However, this type of process class differed depending on the cohort. Responder patients showed enrichment of the pathways relating to IFNy, and also processes relating to T cell biology; both processes are known to constitute antiviral response mechanisms. In the case of the non-responder patients, there is a greater presence of processes relating to phagocytic capacity. Similarly, this group of patients show an increased amount/greater abundance of immune cell migration processes. The majority of these (diapedesis, monocyte adhesion, leukocyte extravasation, etc.) contain PECAM1, which appears expressed in all the samples of non-responder patients. The non-responder patients also showed an increased expression of proteins relating to increased permeability of the vessels.
3. ***MSCs regulatory signaling pathways.*** Differences were detected in the pathways associated with negative and positive regulation of MSC migration in both patient cohorts. These include the HGF signaling, tachykinin signaling, Hippo signaling, NOTCH signaling, Wnt signaling and MAPK signaling pathways.
4. ***Cell-cell adhesion.*** Both patient cohorts showed differences in gene groups relating to cell-cell adhesion: the responders in desmosomes and communicating junctions; whereas the non-responder patients mostly showed processes relating to cytoskeleton reorganization.
5. ***Mitosis.*** A large number of gene groups relating to mitosis appeared overexpressed in the MSCs of responder patients, compared with the MSCs of non-responder patients. Further, the samples from non-responder patients showed overexpression of the gene set that defines the regulation of stem cell proliferation.
6. ***Metabolism.*** Both patient cohorts showed differences in genes relating to metabolism, mainly glycogen, proteins and lipid metabolism. Both groups of patients showed increased glycogen metabolism, which occurred either by direct catabolism (mainly represented by AGL, which was expressed consistently in responder patients but not in non-responders) or by negative regulation of glycogenesis in non-responders. The samples of responder patients showed enrichment of the genes relating to protein metabolism, including peptidase activity and metabolism of peptides and amino acids. On the other hand, non-responder patients showed an increase in genes relating to lipid metabolism (isoprenoid production, and regulation and production of fatty acids and lipoproteins).
7. ***Stress response.*** Both groups of patients showed increased determined pathways relating to the stress response (organelle stress response; DNA damage induced by phosphorylation, regulation of the DNA damage response and endoplasmic reticulum stress).
*8. **Bone marrow fibrosis.*** The samples of non-responder patients showed an enrichment of genes relating to bone marrow fibrosis when compared with responder patients, including angiogenic factors (VEGFA, VEGFB, PDGFA), proinflammatory cytokines (IL 1A and IL 1B) and extracellular matrix remodeling factors (MMP14).

Next, an evaluation was made of whether the proteins that were differentially expressed in the secretome of the MSCs of responder and non-responder patients formed part of the processes that had appeared enriched in the GSEA (p-value < 0.01). In this respect, it was seen that 27 of the 127 candidates participated in at least one of the enriched gene sets (12 out of 81 in the responder patients and 9 out of 59 in non-responder patients). A total of 17 proteins that were differentially expressed in the secretome of the MSCs between responder and non-responder patients formed part of the enriched sets in the samples of responder patients. However, 15 proteins that were differentially expressed and present in the secretome of both patient groups formed part of enriched protein sets in non-responder patients.

### Example 2.3. Biological systems approximation analysis

The object of the first analysis or approximation was simply to identify those genes or proteins that were expressed with greater or lesser intensity in each patient group. Next, a more in-depth analysis was carried out of the processes that could be most relevant in the efficacy or activity of Celyvir: those that were related to the immune response to the virus and to viral replication. To do this, the candidates were evaluated using mathematical analyses of biological systems and artificial neural networks (ANNs).

The ANN model evaluates the relationships that exist between sets of regions within the network or matrix generated (in the present case, genes and/or proteins), providing a predictive score which quantifies the probability of the existence of functional relationships or connections between the regions evaluated (in the present case, the sets of genes/proteins evaluated). Each score is associated with a p-value that indicates the probability of the result being statistically significant. Depending on the value of the p-value, groups of proteins are defined that have:
- A **strong relationship** with the processes studied at that time if the protein set has a strong or medium-strong predictive relationship with any of the sub-processes used for the characterization (p-value < 0.05).
- A **medium relationship** with the processes studied at that time if said processes have at least a medium value relationship with any of the sub-processes used for the characterization (p-value < 0.075 - 0.25).
- A **weak or non-existent relationship** with the processes studied at that time if said processes have a low or non-existent relationship with the rest of the sub-processes used in the characterization (p-value < 0.25).

Given the considerable extent of the full analysis, only the most relevant results obtained for the two above-mentioned processes are detailed.

### Example 2.4. Differential genes obtained in the transcriptome analysis

A total of 44 differentially expressed genes detected in the transcriptome of the MSCs infected with the oncolytic adenovirus ICOVIR-5 from both responder and non-responder patients showed a strong relationship with the following processes:
- Immune response to the virus (29 proteins)
- Viral replication (26 proteins)

For the immune response to the virus process, a total of five genes coding for the same number of proteins were strongly related to at least three different sub-processes involved in this overall process: TRAF3, MAVS, PCBP2, FASN and NDRG1 **(Table 4). Table 4** shows genes differentially expressed by the patients for the immune response to the virus process. The genes that appear in the list showed a strong relationship to said process. Detailed from left to right are the name of the gene; its abundance in the responder patient group; if the gene has an effector role in the process described; the value of the ANN analysis score; and, finally, a description of the specific sub-processes in which said gene intervenes.

**Table 4**

| **ID** | **In resnponders** | **Effector** | **ANN score** | **Related processes** |
|---|---|---|---|---|
| **TRAF3** | Decreased | Yes | 84.19 | Regulation of defense response to the virus |
| | | Yes | 74.32 | Viral pattern-recognition receptor |
| | | Yes | 84.18 | Toll-like receptor signaling |
| | | No | 75.06 | Regulation of Toll-like receptor signaling |
| | | Yes | 74.46 | Type IIFN response |
| | | Yes | 79.08 | Regulation of type I IFN production |
| | | No | 74.15 | Regulation of T cell differentiation |
| | | No | 77.06 | Virus entry into host cell |
| **MAVS** | Decreased | Yes | 90.73 | Regulation of defense response to the virus |
| | | Yes | 77.38 | Defense response to the virus |
| | | Yes | 77.67 | Regulation of signaling pathway mediated by type I IFN |
| | | Yes | 88.63 | Regulation of type I IFN production |
| | | Yes | 92.29 | Regulation of chemokine production |
| **PCBP2** | Decreased | Yes | 82.50 | Regulation of defense response to the virus |
| | | Yes | 81.04 | Defense response to the virus |
| | | Yes | 82.13 | Response to type I IFN |
| | | Yes | 82.46 | Regulation of type I IFN production |
| **FASN** | Decreased | No | 78.50 | Toll-like receptor signaling |
| | | No | 77.39 | Regulation of type I IFN production |
| | | No | 74.14 | Response to IFN-gamma |
| **NDRG1** | Decreased | No | 77.28 | Response to type I IFN |
| | | No | 78.37 | Regulation of the adaptive immune response |
| | | No | 74.30 | Leukocytic differentiation |
| | | No | 81_{.}59 | DNA replication |

For the viral replication process, a total of eight genes coding for the same number of proteins were strongly related to at least two sub-processes involved in this overall process: CHMP2A, CLEC5A, XBP1, BMPR1B, MAVS, RAB29, TRAF3 and MEMO1 **(Table 5). Table 5** shows genes differentially expressed by the patients for the viral replication process. The genes that appear in the list showed a strong relationship to said process. Detailed from left to right are the name of the gene; its abundance in the responder patient group; if the gene has an effector role in the process described; the value of the ANN analysis score; and, finally, a description of the specific sub-processes in which said gene intervenes.

**Table 5**

| ID | In responders | Effector | ANN score | Related processes |
|---|---|---|---|---|
| **CHMP2A** | Increased | No | 75.01 | Viral cycle |
| | | Yes | 77.50 | Regulation of the viral cycle |
| | | Yes | 88.52 | Viral assembly |
| | | Yes | 85.04 | Regulation of viral release from the host cell |
| **CLEC5A** | Increased | Yes | 80.38 | Viral cycle |
| | | Yes | 85.74 | Virus entry into host cell |
| **XBP1** | Increased | Yes | 81.54 | Regulation of immunity mediated by immunoglobulins |
| | | No | 74.49 | Viral cycle |
| | | No | 77.73 | Regulation of translation |
| **BMPR1 B** | Increased | No | 75.93 | Viral cycle |
| | | No | 75.53 | DNA replication |
| **MAVS** | Decreased | Yes | 82.30 | Regulation of viral cycle |
| | | No | 77.23 | Cell response to virus |
| **RAB29** | Decreased | Yes | 80.00 | Viral cycle |
| | | No | 78.42 | Regulation of viral transcription |
| **TRAF3** | Decreased | No | 77.06 | Viral entry into host cell |
| | | No | 76.78 | Cell response to virus |
| **MEM01** | Decreased | No | 74.24 | RNA interference |
| | | No | 75.19 | Regulation of viral transcription |
| | | No | 76.01 | Regulation of translation |

### Example 2.5 Validation of the candidates obtained in the transcriptome studies

Once the molecules involved in the different biological behavior of the ATMP between both groups of patients regarding cell response to infection and permissivity for adenoviral replication were identified, said molecules were validated. To do this, an independent cohort of patients treated with the Celyvir ATMP was used.

### Example 2.6. Validation of candidate genes responsible for the differences in the innate cellular response to infection

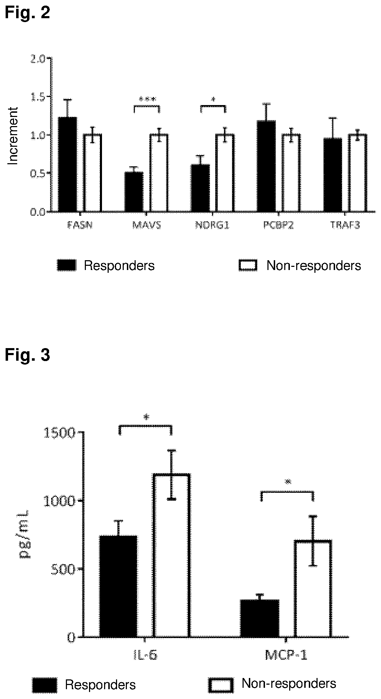

A quantitative PCR was carried out for the FASN, MAVS, NDRG1, PCBP2 and TRAF3 genes. The results revealed that only MAVS and NDRG1 met this condition **(****Fig. 2****).**

### Example 2.7. Quantification of secretome candidates

The other two analytes (IL-6 and MCP-1) showed significantly lower values in the non-responder group **(****Fig. 3****).**

The validation results obtained in the study of the second cohort are consistent with what is known about MAVS signaling. The relationship between IL6 and CCL2 and MAVS has been described (Seth RB, Sun L, Ea C-K, Chen ZJ. Identification and characterization of MAVS, a mitochondrial antiviral signaling protein that activates NF-κB and IRF3. Cell 2005; 122: 669-682) (Jiang C, Lin X. Regulation of NF-κB by the CARD proteins. Immunol Rev 2012; 246: 141-153), such that MAVS regulates the expression of IL6 and CCL2 via the NF-κB pathway. The inability to produce interferon in the context of an antiviral response in knockout (deficient) mice for MAVS (-/-) has been demonstrated (Sun Q, Sun L, Liu H-H, Chen X, Seth RB, Forman J et al. The specific and essential role of MAVS in antiviral innate immune responses. Immunity 2006: 24: 633-642). This role of MAVS has been detected in immune cells (mainly macrophages and dendritic cells) (Di Fiore IJM, Holloway G, Coulson BS. Innate immune responses to rotavirus infection in macrophages depend on MAVS but involve neither the NLRP3 inflammasome nor JNK and p38 signaling pathways. Virus Res 2015; 208: 89-97) (Roe K, Giordano D, Young LB, Draves KE, Holder U, Suthar MS et al. Dendritic cell-associated MAVS is required to control West Nile virus replication and ensuing humoral immune responses. PloS One 2019; 14: e0218928) (Vazquez C, Horner SM. MAVS Coordination of Antiviral Innate Immunity. J Virol 2015; 89: 6974-6977), but as far as is known, this is the first time the regulatory role of MAVS in mesenchymal cells has been described and confirmed.

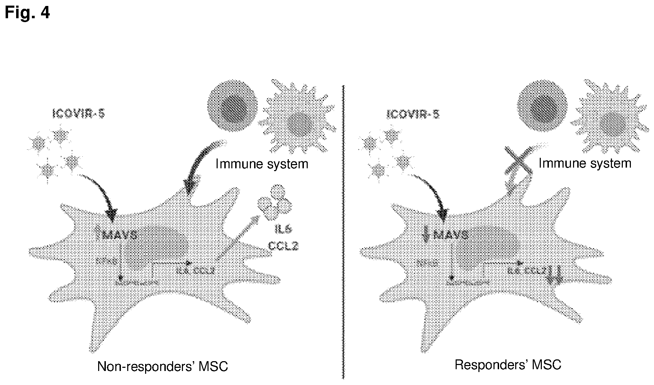

In sum, the low expression of these two genes and these two proteins (resulting from lower signaling via the first) in responder patients indicates lower signaling of the intracellular presence of ICOVIR-5, or delayed signaling, with significantly less production of immune response mediators. In any event, the MSCs of responder patients show under-stimulation of the immune system so that said immune system develops an antiviral response and therefore an immune response to Celyvir. This allows the mesenchymal cells to migrate to the tumor sites without being neutralized by the patient's immune system at the time ICOVIR-5 is being replicated inside the MSCs. A graphic illustration of the hypothesis is that shown below **(****Fig. 4****).**

## Claims

1. Substantially pure population of mesenchymal stem cells, wherein the mesenchymal stem cells constitute at least 80% of the total cell population, **characterized in that** expression of the MAVS gene or other genes regulated by said gene is inhibited.

2. Substantially pure population of mesenchymal stem cells according to claim 1, wherein the mesenchymal stem cell is derived from bone marrow, placenta, umbilical cord, amniotic membrane, menstrual blood, peripheral blood, salivary gland, skin and prepuce, synovial fluid, amniotic fluid, endometrium, adipose tissue, umbilical cord blood and/or dental tissue.

3. Substantially pure population of mesenchymal stem cells according to either claim 1 or claim 2 for use as transporters or vehicles for therapeutic agents.

4. Composition which comprises: i) at least one cell according to either claim 1 or claim 2 and ii) at least one antigenic substance.

5. Composition according to claim 4, wherein the antigenic substance is an intracellular parasite.

6. Composition according to either claim 4 or claim 5, wherein the intracellular parasite is an oncolytic virus.

7. Composition according to any one of claims 4 to 6, wherein the intracellular parasite is ICOVIR-5.

8. Pharmaceutical composition which comprises a composition according to any one of claims 4 to 7 and pharmaceutically acceptable vehicles, adjuvants and/or excipients.

9. Composition according to any one of claims 4 to 7, or pharmaceutical composition according to claim 8, for use as a medicine.

10. Composition according to any one of claims 4 to 7, or pharmaceutical composition according to claim 8, for use in the treatment of solid tumors.

11. Composition for use in the treatment of solid tumors according to claim 10, wherein the solid tumor is brain cancer, lung cancer, renal cancer, ovarian cancer and/or liver cancer.

12. *In vitro* method for the production of a substantially pure population of modified mesenchymal stem cells which comprises:
a. Obtaining mesenchymal stem cells from an isolated sample;
b. Culturing the mesenchymal stem cells;
c. Inhibiting expression of the MAVS gene, or other genes regulated by said gene, in the cultured cells.

13. Method according to claim 12 which also comprises infecting the cells with an intracellular parasite.

14. Kit which comprises:
a. At least one mesenchymal stem cell;
a. A system capable of inhibiting expression of the MAVS gene, or other genes regulated by said gene.

15. Kit according to claim 14, wherein the system capable of inhibiting expression of the gene is a small interfering RNA or gene therapy vector.
